# EUROPEAN PATENT APPLICATION

(11) **EP 4 659 656 A1**
(43) Date of publication of application: **10.12.2025**
(21) Application number: 24211697.8
(22) Date of filing: 08.11.2024
(51) Int. Cl.: A61B 5/00

(54) **APPARATUS AND SYSTEM FOR SENSING PROPERTIES OF BIOLOGICAL TISSUE**

(30) Priority: 07.06.2024 EP 24180942
(71) Applicant: iThera Medical GmbH, 81379 München (DE)
(72) Inventor: Konradl, Josef, 85235 Odelzhausen (DE); Leisching, Patrick, 81247 München (DE)
(74) Representative: Wallinger Ricker Schlotter Tostmann

(57) **Abstract**

The disclosure relates to an apparatus for sensing properties of biological tissue, the apparatus comprising a probe, in particular a handheld probe, the probe comprising: a plurality of laser radiation emitting elements configured to emit pulses of laser radiation, the laser radiation having a predefined wavelength and the pulses of laser radiation having a predefined pulse width and predefined pulse repetition rate, an exit section, in particular an opening or window, through which the emitted pulses of laser radiation can exit the probe and impinge on the biological tissue, and at least one detector, in particular including piezo, CMUT or PMUT elements with or without preamplifier, configured to detect signals emanating from the bio-logical tissue in response to the impinging pulses of laser radiation and to convert the detected signals into detector signals from which one or more properties of the biological tissue are derivable, and is characterized in that the laser radiation emitting elements are distributed over a two-dimensional sur-face, whereby for the predefined wavelength, pulse width and pulse repetition rate a radiation energy of the emitted pulses of laser radiation at or close to the exit section of between 1 µJ/mm² and 50 µJ/mm², in particular over an area of 1 to 200 mm² and/or at an emission angle of 15° to 40°, in particular 15° to 35°, is obtained.

## Description

The present disclosure relates to an apparatus and a system for sensing properties of biological tissue according to the independent claims.

Currently, routine clinical use and Point-of-Care (PoC) optoacoustic imaging (herein also referred to as "OA" or "OAI") devices are non-existent because current scanner technology is bulky, expensive, power consuming (>1000 W) and complicated to operate. The state-of-the-art laser sub-component (including electronics) amounts for circa 50% of the total cost, size and power consumption. Laser technologies based on high power lasers (e.g., Nd:YAG+OPO), LEDs, or photodiodes have a low wall-plug efficiency and therefore require active cooling, consume a vast amount of energy, are bulky, make the examination room hot and noisy (air conditioning needed), and necessitate wearing safety goggles that are impractical for the clinician and uncomfortable for the patient. The electronics subsystem amounts for circa 25% of the total cost. Dedicated electronics (referred to as "DAQ" for "Data Acquisition device") with high throughput acquisition hardware are required to integrate the ultrasound and laser sub-components: triggering and transmission of the excitation signals, reception of the generated OA and reflected ultrasonic (herein also referred to as "US") acoustic pressure waves. Additionally, a high-performance graphic card is needed to reconstruct high quality OA images in real-time. The piezoelectric (i.e., ceramics, crystals, polymers and piezocomposite) ultrasound transducers sub-component amounts for circa 15% of the total cost.

Currently, the classification of OAI devices according to the prior art as laser class 3B/4 devices is cumbersome for any routine clinical application beyond radiology research-driven hospitals, in particular because a laser safety room is needed, as is a laser safety officer. Additionally, the doctor and the patient have to wear laser protecting goggles during the examination.

It is an object of present disclosure to provide an improved apparatus, preferably including a handheld device (also referred to as "probe"), and system for optoacoustic sensing of properties, in particular imaging, of biological tissue, wherein laser safety requirements and/or standards are fulfilled or met, in particular without necessitating wearing safety goggles.

The object is achieved by an apparatus and a system according to the independent claims. Preferred and/or alternative aspects or embodiments are subject of the dependent claims and/or the following description.

According to a first aspect, an apparatus for sensing properties of biological tissue comprises a probe, in particular a handheld probe, the probe comprising: a plurality of laser radiation emitting elements configured to emit pulses of laser radiation, the laser radiation having a predefined wavelength and the pulses of laser radiation having a predefined pulse width and predefined pulse repetition rate; an exit section, in particular an opening or window, through which the emitted pulses of laser radiation can exit the probe and impinge on the biological tissue; and at least one detector, in particular including ceramic piezo, CMUT or PMUT elements with or without preamplifier, configured to detect signals emanating from the biological tissue in response to the impinging pulses of laser radiation and to convert the detected signals into detector signals from which one or more properties, in particular image(s), of the biological tissue are derivable. The laser radiation emitting elements are spatially distributed, preferably over a two-dimensional surface, whereby for the predefined wavelength and/or pulse width and/or pulse repetition rate a radiation energy, also referred to as "radiant exposure" or "fluence", of the emitted pulses of laser radiation at or close to the exit section of between 1 µJ/mm² and 50 µJ/mm² is obtained. Preferably, the mentioned radiation energy range is obtained over an area at or close to the exit section of 1 to 200 mm² and/or at an emission angle, also referred to as "divergence", of 10° to 45°, preferably 15° to 35°.

According to a second aspect, a system for optoacoustic imaging of an object comprises an apparatus according to the first aspect and a computer system, in particular a mobile computer device and/or a computer workstation and/or a cloud computing system, located outside the apparatus, in particular outside the probe, and being in a data communication with the apparatus, the computer system being configured to process the detection signals and/or to derive one or more properties of the biological tissue from the detection signals and/or to reconstruct an image of the biological tissue based on the detection signals and/or to further process an image of the biological tissue which has been reconstructed from the detection signals.

Aspects of the present disclosure are preferably based on the approach of providing a plurality of laser radiation emitting elements, which are given, e.g., by exit faces of a plurality of fibers and/or by a plurality of laser diodes such as VCSELs, within the handheld probe and spatially arranging and/or distributing the laser radiation emitting elements within the handheld probe, in particular over a two-dimensional area, such that the laser radiation emitted by the individual laser radiation emitting elements overlaps to form a total laser radiation beam which thereby has, for a predefined wavelength, pulse width and pulse repetition rate of the laser radiation, a radiation energy of between 1 µJ/mm² and 50 µJ/mm² when exiting the probe via an exit section of the probe. In this way, laser class 1 or 3R can be fulfilled, while at the same time the emitted radiation and/or the radiation exposure of the tissue to be investigated is strong enough such that, e.g., optoacoustic and/or optical sensing is possible, in particular in that signals emanating from the biological tissue in response to the impinging pulses of laser radiation can be reliably detected and converted into detector signals from which one or more properties of the biological tissue are derivable. In other words, aspects of present disclosure are preferably based on the approach of using the concept of an "extended source" to get classification of laser class 1 or 3R, in particular in accordance with requirements specified in the standard of the International Electrotechnical Commission IEC 60825-1. To achieve this goal, the radiation sources given by a VCSEL array or a multi-fiber fiber delivery (also referred to as "light guide") are designed to fit into the framework of the extended source.

Other than point sources, these extended light sources have a certain dimension d_{source} and an opening angle leading to considerably higher continuous-wave (CW) and pulse power limits: the human eye cannot focus all photons to a single spot on the retina, only to a size of d_{image}. According to preferred aspects of present disclosure, VCSEL arrays with a plurality, e.g. thousands, of individual VCSELs and/or a multi-mode fiber delivery with a plurality, e.g. hundreds, of fibers can be designed (arranged and/or controlled and/or supplied with pulsed radiation having particular properties, respectively) in a way that laser class 1 or 3R is achieved for an optoacoustic handheld device (probe), apparatus and system, respectively.

For example, it was found that for the far field of a customized 2x2 mm² VCSEL array a factor of 16 in the pulsed output power is the headroom for laser class 1, and a factor of 80 for class 3R. The classification depends on the density of the individual VCSELs on the array, the shape of the individual VCSELs, the divergence angle of the VCSEL light sources and the operating CW and peak power. Due to the lower peak power, a higher repetition is preferably used resulting in a higher CW power: This CW power is adjusted via the repetition rate to eye safety limits for CW.

Likewise, the fiber delivery coupled with a solid-state laser system, e.g., a YAG-OPO system, can be engineered in a similar way so that a class 3R operation is achievable. Due to a lower repetition rate, the CW limit for eye safety is not necessarily reached, but the peak power limit is achieved. The maximum allowed output power can be engineered by at least one of the following: the number of fibers, their spatial arrangement, the fill factor, the core diameter and type of fiber (multi-mode/single-mode) of the fiber, diffusers before and/or after the fiber, light shaping optics (e.g. refractive or diffractive optical elements) before the fiber, and the numerical aperture (NA) of the individual fibers at the exit face. The higher the NA, the larger the divergence angle of the photon beam is, wherein the angle can be optionally corrected by a lens without affecting the "extended source" image. In preferred implementations in OAI devices the power levels are more than a factor of 10 above class 3R.

To the best inventors' knowledge, there is currently no implementation of a portable optoacoustic handheld device (probe) with laser class 1 or 3R for real-time imaging medical applications (frame rate > 5 Hz). Further, current implementations require a large electronic infrastructure to support the YAG-OPO, LED, VCSEL or diode laser implementations, the power consumption (laser and electronic driver) is between 100 and 1000 W, a pure handheld device requires less than 15 W and a maximum surface temperature of 43°C. The complex solid state and laser diode implementations (with or without fiber delivery) exceed the eye safety thresholds of IEC 60825-1 or ANSI Z136.1. Further, an LED being no laser does not fall under IEC standard but cannot be fiber-coupled and has a low wall-plug efficiency that prohibits integration of the electronic infrastructure in the handheld device due to thermal heating issues.

In summary, present disclosure provides an improved apparatus including a probe, in particular a handheld probe, and an improved system for optoacoustic sensing, in particular imaging, of biological tissue wherein laser safety requirements and/or standards, in particular laser class 1 or 3R, are met or complied with, so that the wearing of safety goggles or protective eyewear is no longer required.

According to a preferred first embodiment, the apparatus further comprises: a laser source, in particular an Nd:YAG pumped optical parametric oscillator (OPO) and/or at least one edge-emitting diode laser or an edge-emitting diode laser array and/or at least one VCSEL or a VCSEL array, configured to emit pulsed laser radiation having the predefined wavelength, the predefined pulse width and the predefined pulse repetition rate; and a fiber bundle comprising a plurality of single fibers each having an entry face and an exit face, wherein the fibers are arranged, in particular bundled, in such a way that, at a proximal end of the fiber bundle, the laser radiation emitted by the laser source is or can be coupled into the entry faces of the fibers and, at a distal end of the fiber bundle, the exit faces of the fibers are spatially distributed, preferably over the two-dimensional surface, and thereby constitute the laser radiation emitting elements.

Preferably, the fiber bundle comprises at least 300, in particular at least 400, 500 or up to 1500, single fibers.

Preferably, the single fibers have a diameter of between 10 and 500 µm, in particular between 100 and 300 µm or between 150 and 250 µm or between 180 and 220 µm or approximately 200 µm.

Preferably, the exit faces of the fibers are distributed or arranged over the two-dimensional surface in such a way that on average between 4 and 100, in particular between 10 and 50 or between 15 and 30 or approximately 20, exit faces of the fibers are provided per square millimeter (mm²) of the two-dimensional surface.

By means of the above aspects, alone or in combination, the desired radiation energy range at or close to the exit section of the probe can be obtained in a both reliable and simple manner by defining properties, in particular geometrical properties and/or the numerical aperture, of the fiber bundle and/or of the single fibers, in particular without requiring too many single fibers or too small diameters of the single fibers.

Preferably, the repetition rate is between 10 Hz and 100 Hz, in particular between 20 Hz and 30 Hz, in particular if the laser source is an Nd:YAG pumped optical parametric oscillator (OPO), or between 10 Hz and 10 kHz or 20 kHz, preferably between 100 Hz and 10 kHz, in particular if the laser source is a VCSEL array.

Alternatively or additionally, the predefined wavelength is between 650 nm and 1200 nm and/or the predefined pulse width is between 1 ns and 200 ns, in particular between 4 ns and 20 ns if the laser source is an Nd:YAG-OPO or between 5 ns and 200 ns if the laser source is an edge-emitting diode laser array and/or a VCSEL array, and/or the predefined pulse repetition rate is between 10 Hz and 100 Hz if the laser source is an Nd:YAG-OPO or between 100 Hz and 10 kHz if the laser source is an edge-emitting diode laser (array) and/or a VCSEL (array), and/or the exit faces of the fibers are distributed over the two-dimensional surface in such a way that on average between 10 and 100 exit faces of the fibers are provided per square millimeter (mm²) of the two-dimensional surface, whereby a radiation energy of the emitted pulses of laser radiation at or close to the exit section of between 1 µJ/mm² and 50 µJ/mm² is obtained. In this way, the desired radiation energy range at or close to the exit section of the probe can be reliably and simply obtained by providing specific properties of the emitted laser radiation, in particular a specific wavelength range of the radiation and/or pulse width and/or pulse repetition rate of the pulses, and/or by providing a specific spatial density of the fiber exit faces, preferably with a defined numerical aperture.

According to a preferred second embodiment, the plurality of laser radiation emitting elements is given by a plurality of vertical-cavity surface-emitting lasers (VCSELs) which are spatially distributed over the two-dimensional surface and configured to emit the pulses of laser radiation.

Preferably, the plurality of laser radiation emitting elements is given by at least 1000, in particular at least 1500 or at least 2000, preferably 2200, VCSELs and/or the two-dimensional surface has a size of 0.5 to 4 mm².

Preferably, the VCSELs are distributed over the two-dimensional surface in such a way that on average between 100 and 1000, in particular between 300 and 600, VCSELs are provided per square millimeter (mm²) of the two-dimensional surface.

Preferably, the repetition rate is between 100 Hz and 10 kHz, in particular between 500Hz and 2 kHz.

Preferably, the predefined wavelength is between 650 nm and 1200 nm, the predefined pulse width is between 1 ns and 200 ns, the predefined pulse repetition rate is between 100 Hz and 10 kHz, the VCSELs are distributed over the two-dimensional surface in such a way that on average between 100 and 1000 VCSELs are provided per square millimeter (mm²) of the two-dimensional surface, whereby a radiation energy of the emitted pulses of laser radiation at or close to the exit section of between 1 µJ/mm² and 50 µJ/mm² is obtained. Preferably, the divergence angle of the VCSEL is a design parameter of the individual VCSEL mesas on the array.

The second embodiment including its preferred aspects, alone or in combination, described above provides an alternative to the first embodiment, wherein the laser radiation emitting elements, which are spatially distributed or arranged over the two- or three-dimensional surface or area to achieve the desired radiation energy ("radiant exposure" or "fluence") at or close to the exit section, are VCSELs instead of laser radiation emitting ends ("exit faces") of optical fibers. Therefore, the above-mentioned advantages of the first embodiment and its preferred aspects apply accordingly to the second embodiment and its preferred aspects, alone or in combination, in particular in that the desired radiation energy range at or close to the exit section of the probe can be reliably and simply obtained by defining geometrical properties of the VCSELs and/or their arrangement, in particular without requiring too many VCSELs and/or too small dimensions of the VCSELs, and/or by providing specific properties of the emitted laser radiation, in particular a specific wavelength range of the radiation and/or pulse width and/or pulse repetition rate of the pulses, and/or by providing a specific spatial density of the VCSELs, preferably within a defined divergence angle.

Preferably, an irradiance of the emitted pulses of laser radiation at or close to the exit section is between 0.5 mW/mm² and 5 mW/mm², in particular between 1.5 mW/mm² and 3 mW/mm². It is noted that this power level (i.e. the irradiance) preferably depends on a maximum CW power exposition (MPE) threshold, which is preferably specified by IEC 60825-1, at the particular wavelength of the laser radiation. In other words, the irradiance of the emitted pulses of laser radiation at or close to the exit section is preferably determined by considering an MPE threshold specified by IEC 60825-1 at the particular wavelength.

Preferably, the emitted pulses of laser radiation exit the probe at the exit section at an emission angle (divergence) of between 15° and 35° relative to the perpendicular of the exit section.

Basically, the preferred emission angle (divergence") of 10° to 45°, preferably 15° to 35°, can be achieved with or without an additional optical element, such as a lens, which may be optionally provided to correct for the VCSEL light emission angle. For example, if the divergence of a VCSEL or VCSEL array is 35°, a lens may be provided to shape the divergence from 35° to a, e.g., 10° opening angle.

Preferably, the apparatus may further comprise at least one lens, in particular at least one focusing lens, such as a spherical or cylindrical lens or a Fresnel lens, which is preferably arranged between the laser radiation emitting elements, in particular the exit faces of the fibers or the VCSELs, and the exit section of the probe and configured i) to focus the laser radiation emitted by the laser radiation emitting elements to a focus point or focus area which is located within the probe and/or at or close to the exit section of the probe and/or ii) to collimate the laser radiation emitted by the laser radiation emitting elements. This embodiment may be particularly preferred in cases where, for example, the VCSELs have larger emission angles (divergence) of, e.g., 30° to 45°. In such cases, it is preferred to focus the laser radiation at or close to the exit section of the probe and/or to an acoustic couplant provided between the exit section and the object, preferably by means of a cylindrical lens arranged in parallel to the VCSEL array. Alternatively or additionally, it may be preferred that the lens creates a focus within the probe. This would allow a smaller illumination spot while still having a large divergence angle at the output (exit section). In other words, radiation emitted by a VCSEL (or VCSEL array) that is collimated with a lens can have a small or narrow illumination spot, stripe or row at the skin surface. Radiation emitted by a VCSEL or VCSEL array which is focused within the probe and then further diverges to the same spot size, stripe or row width, respectively, at the skin surface will have a larger divergence angle. Alternatively or additionally, more than one lens, in particular at least two lenses, may be provided such that one lens collimates the laser radiation and another lens increases the divergence angle very near the skin surface. Alternatively or additionally, it may be preferred that the at least one lens focuses the laser radiation emitted by a VCSEL (or VCSEL array) to a small or narrow spot, stripe or row at the exit section, which is preferably given by a membrane. Preferably, near the membrane or integrated into the membrane a diffusor may be provided which (further) increases the divergence of the light after exiting the probe.

Preferably, in a laser class 1/3R device or apparatus, the VCSEL arrays (lasers) are an extended source and not all of the VCSEL mesas in the array account for the power limits. One alternative implementation uses the laser in a cart system, apparatus and/or device and a multi-fiber delivery to achieve an extended laser source in the handheld. The power can be limited by the diode driving unit using open or closed-loop control electronics and/or an appropriate dimensioning of the drive electronics.

Preferably, in a laser class 3R handheld device or apparatus, "chip tuning" of a class 1 device, the power limitation of the laser drivers of the class 1 device are removed. One alternative implementation uses the laser in the cart system and a multi-fiber delivery to achieve an extended laser source in the handheld. The extended laser source is optionally covered by an optical diffuser element. Class 3R allows 5x class 1 power limits.

Preferably, for quantification of oxygenation in tissue, reconstruction algorithms, such as *Efficient Deep Model-Based Optoacoustic Image Reconstruction* as described by C. Dehner and G. Zahnd in arXiv:2408.07109 [eess.IV] (https://doi.org/10.48550/arXiv.2408.07109), can be used to allow for high frame rate > 5 Hz.

Preferably, the illumination unit additionally comprises a plurality of optical fibers configured to deliver light from a light source external to the housing of the handheld device.

Preferably, a first radiation source in the optical output is configured to be within the limits of a class 1 or 3R laser device according to IEC60825-1, wherein the controller is configured to modulate the power of the illumination unit based on the position and/or distance and/or movement information derived by the controller.

Preferably, in the illumination unit the optical output of the handheld device is an extended source.

Preferably, the illumination unit contains an optical diffuser.

Preferably, the illumination unit contains an additional lens for a divergence >25°.

Preferably, in the information the parameter of the object is oxygenation of hemoglobin, a ratio of different photoabsorbers, a concentration of a photoabsorber, etc.

Preferably, the at least one controller is configured to reconstruct optoacoustic images from the recorded signals at a frame rate above 5 Hz.

Preferably, the at least one controller is configured to reconstruct ultrasound images from the recorded signals at a frame rate above 5 Hz.

Preferably, in the illumination unit the extended source of radiation is an array of optical emitters such as a VCSEL array or a multi-fiber delivery or the properties of an extended source are achieved by use of refractive or diffractive optical elements or a light scattering element (optical diffuser).

Preferably, the illumination unit is and/or comprises a class 1/3R laser.

Optionally, a pulse energy calibration can be performed: A (photo)diode is provided specifically for assessing the pulse energy from the laser. This is not necessarily needed and/or dispensable for VCSELs because of their inherent stability and low rate of degradation. Alternatively or additionally, to sense if the VCSELs (or YAG-OPO) are firing or not (e.g., for safety, not calibration), a current-sensing circuit may be provided instead of measuring light output by means of a (photo)diode.

Further additional or alternative aspects, advantages, features and examples of the present disclosure will be apparent from the following description of following figures, wherein:
- Fig. 1: shows a) an example of an apparatus and/or system for optoacoustic sensing and/or imaging, and b) an example of a handheld device or probe;
- Fig. 2: shows preferred implementations of the extended source concept by providing a) a VCSEL light source in the handheld device, or b) a multi-fiber delivery;
- Fig. 3: a) to c) show an overview on definitions of laser classes according to IEC 60825-1, including laser class 1 and 3R, and optical systems for determining properties, in particular the maximum power exposure (MPE) levels, for a point source vs. an extended light source; and
- Fig. 4: shows preferred details of implementations of the extended light source concept, wherein a) and b) refer to a VCSEL subsystem, and c) refers to transmitting light from a laser source via a multi-fiber delivery.

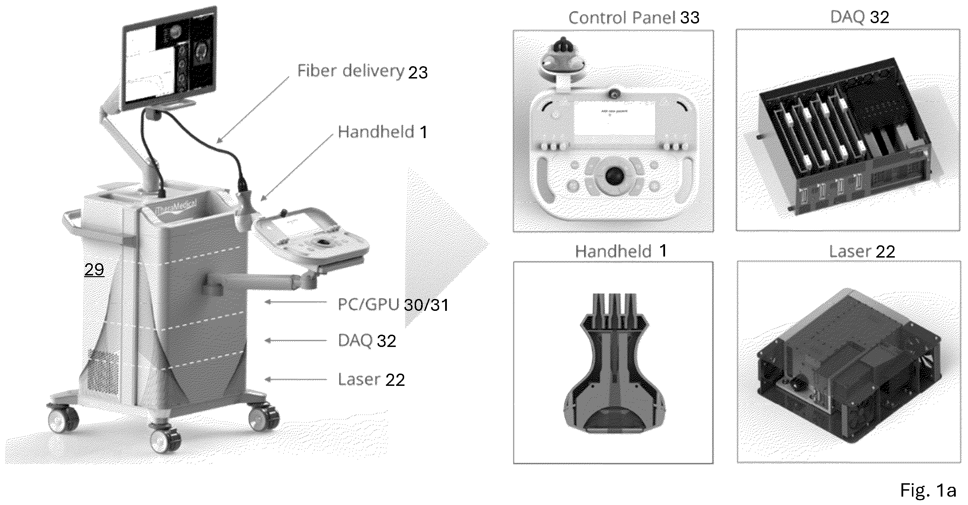

Figure 1a shows a perspective view of an example of an apparatus and system for optoacoustic sensing and/or imaging (left) and an enlarged schematic representation of some of its components (right). The apparatus and system is preferably configured as a cart-based OAI system comprising a fiber delivery 23 including a plurality of optical fibers for guiding light generated by a YAG-OPO laser 22 and/or by an edge-emitting laser diode bar and/or a VCSEL array (not shown) provided in the cart 29 to a handheld device 1, which is also shown enlarged in the right part of the figure. The system and/or apparatus preferably further comprises a data acquisition unit (DAQ) 32 which is configured to process acoustic waves and/or optical signals which are detected by the handheld device 1, for example by sampling and/or converting the analog signals into digital signals. Further, a controller 30, in particular a computer such as a PC, and/or a graphics processing unit (GPU) 31 is provided for further processing the digital signals, e.g., by deriving properties of a biological tissue from the signals and/or reconstructing an image of the biological tissue based on the signals and/or processing an image of the biological tissue which has been reconstructed from the signals. Further, a control panel 33 may be provided for controlling the system, apparatus and/or handheld device 1, in particular during the examination of a patient.

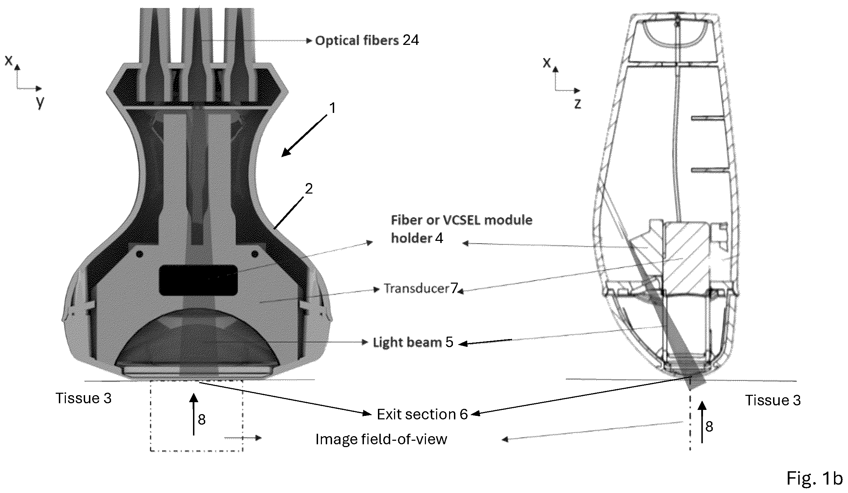

Figure 1b shows a schematic cross-sectional representation of a handheld device 1, preferably of the handheld device 1 shown in figure 1a, which is in contact with a tissue 3, in a front view (left) and side view (right) to illustrate two preferred (alternative) implementations of the concept of an extended light source. It is noted that although both implementations are illustrated by means of the handheld device 1 shown in Figure 1b, it is preferred that a handheld device 1 implements either one or the other of the two implementations.

In a first implementation, light is guided by means of a plurality (e.g. 300 to 1500) of optical fibers 24 of a fiber delivery 23 (see figure 1a) into the handheld device 1 where, preferably at or by means of a fiber holder 4, the light-emitting distal ends of the fibers 24 are arranged such that the resulting total light beam 5 exhibits an opening angle of 15° to 35° and/or has a lateral size which is expanded from approximately 1 mm (at the fiber holder 4) to approximately 1-10 mm when emerging from the handheld device 1 at an exit section 6 and impinging on the tissue 3. Preferably, the single fibers 24 of the fiber delivery 23 have a diameter of between 10 and 500 µm and/or the exit faces of the single fibers 24 are distributed over a two-dimensional surface (which is preferably given by the cross-section of the distal end of the fiber bundle 23, as explained further below) such that, on average, between 10 and 100, in particular between 50 and 70, exit faces of the fibers 24 are provided per square millimeter (mm²). Preferably, for pulsed light having wavelengths between 650 nm and 1200 nm, pulse widths between 1 ns and 200 ns and pulse repetition rates between 10 Hz and 10 kHz, a radiation energy of the emitted light pulses at or close to the exit section 6 of between 1 µJ/mm² and 50 µJ/mm² is obtained in this way. Preferably, the predefined pulse width is between 4 ns and 20 ns if the laser source is an Nd:YAG-OPO or between 5 ns and 200 ns if the laser source is an edge-emitting diode laser (array) and/or a VCSEL (array). Alternatively or additionally, the predefined pulse repetition rate is between 10 Hz and 100 Hz if the laser source is an Nd:YAG-OPO or between 100 Hz and 10 kHz if the laser source is an edge-emitting diode laser (array) and/or a VCSEL (array).

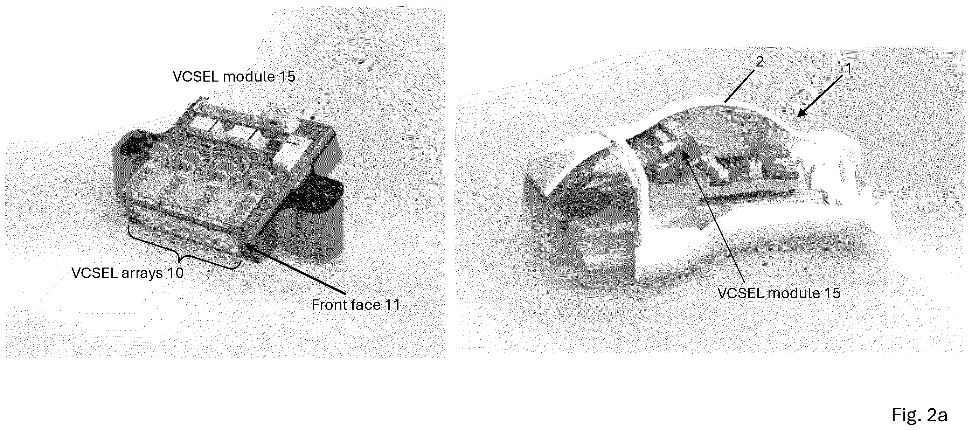

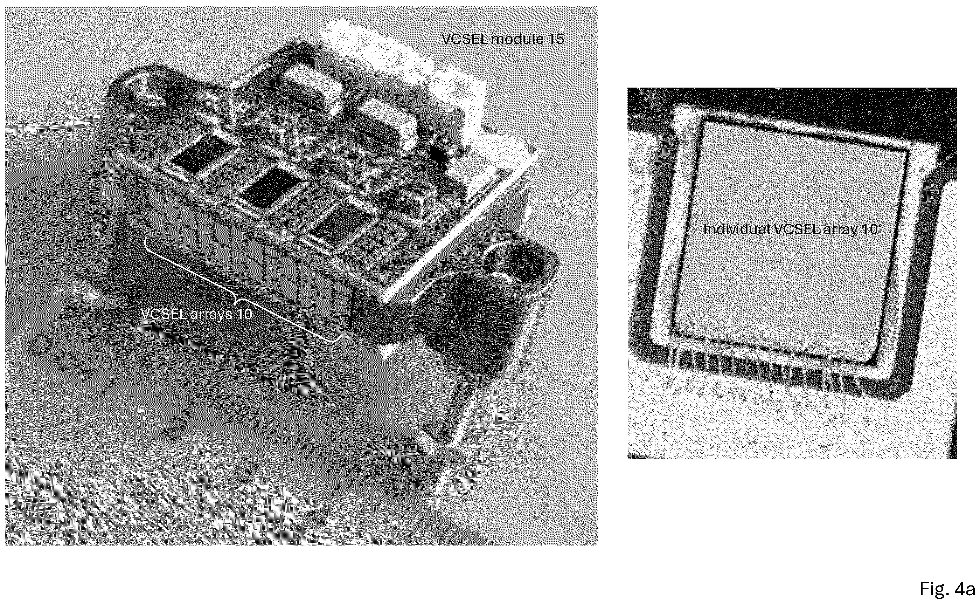

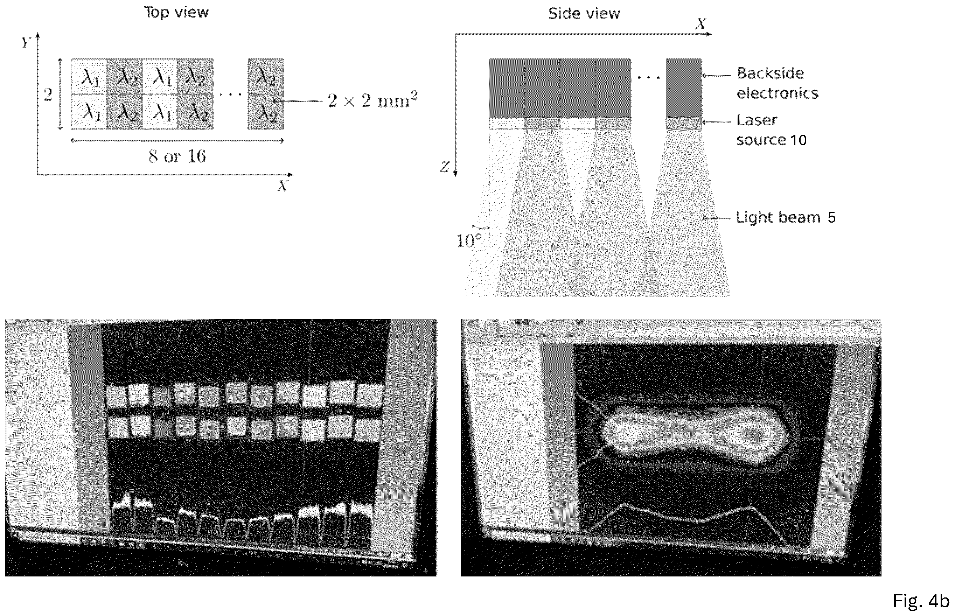

In a second, in particular alternative, implementation the light is generated by means of a VCSEL array comprising a plurality (e.g. 1000 to 2200) of individual VCSEL mesas (not shown, see figures 2a, 4a, 4b) provided at the VCSEL module holder 4, wherein the VCSELs are arranged and/or distributed such that the resulting total light beam also exhibits an opening angle of 15° to 35° and/or has a lateral size which is approximately 1-10 mm when emerging from the handheld device 1 at the exit section 6 and impinging on the tissue 3. Preferably, the VCSELs are distributed over a two-dimensional surface (which preferably given by a front face of a VCSEL module, see below for further details) in such a way that, on average, between 100 and 1000, in particular between 300 and 800, VCSELs are provided per square millimeter (mm²). Preferably, for pulsed light having wavelengths between 650 nm and 1200 nm, pulse widths between 1 ns and 200 ns and pulse repetition rates between 100 Hz and 10 kHz, a radiation energy of the emitted light pulses at or close to the exit section 6 of between 1 µJ/mm² and 50 µJ/mm² is obtained in this way.

In both implementations, the radiation energy of the light 5 emerging from the exit section 6 of the handheld device 1 is low enough to meet the requirements of certain laser safety classes (in particular class 1 or 3R), yet high enough that the radiation exposure of the tissue 3 to be examined is strong enough to enable optoacoustic sensing and/or imaging.

The handheld device 1 further comprises a transducer 7, also referred to as "detector" herein, which is configured to detect acoustic waves 8 emanating from the tissue 3 in response to the impinging light pulses 5 and to convert the detected acoustic waves 8 into detection signals. In the example shown, the detector 7 comprises a plurality of transducer elements, in particular piezo, CMUT and/or PMUT elements with or without preamplifier, which are preferably arranged on a curved, preferably concave, surface.

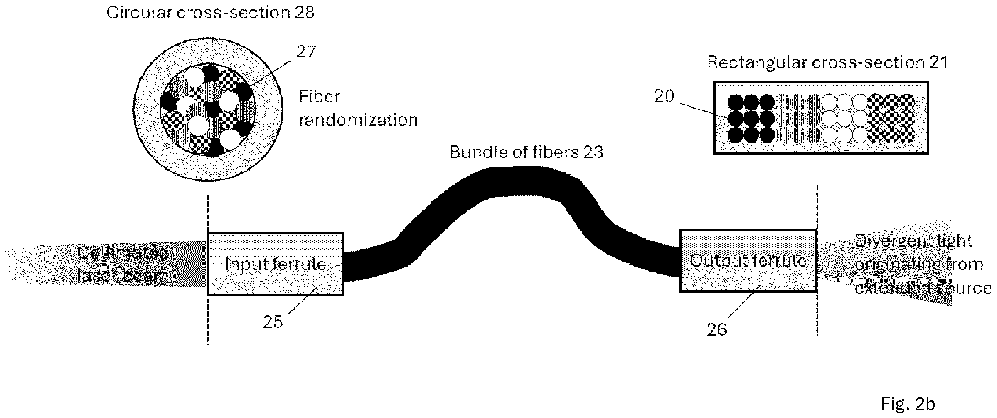

Preferred implementations of the extended source concept are further exemplarily illustrated in figure 2a (with a "direct" integration of a VCSEL light source in the handheld device 1) and figure 2b (with a multi-fiber delivery 23 of light generated by a light source provided in the cart 29, see figure 1a).

Specifically, figure 2a shows perspective views of an example of a VCSEL module 15 (left) including one or more VCSEL arrays 10, and a handheld device 1(with partly transparent representation of the housing 2 for illustration purposes) with an integrated VCSEL module 15 (right). As apparent from figure 2a (left), the VCSEL arrays 10 are provided on a, preferably planar, front face 11 of the VCSEL module 15 so that the plurality of VCSELs (comprised by the VCSEL arrays 10) are spatially distributed over the front face 11, which is therefore considered to be and/or to comprise a "two-dimensional surface" within the meaning of the present disclosure.

Further, figure 2b shows a schematic representation of an example of a fiber delivery system including a plurality of optical fibers which are configured to guide light from a laser source (not shown) into a handheld device (not shown, see figure 1b, 2a), wherein a collimated laser beam enters an input ferrule 25 of a fiber bundle 23 having an essentially circular cross-section 28, over which the single fibers are, preferably randomly, distributed, and exits the fiber bundle 23 via an output ferrule 26 (provided in the handheld device 1, e.g. at the fiber holder 4 (see figure 1b), having an essentially rectangular cross-section 21, over which the exit faces 20 of the single fibers are, preferably evenly/or regularly, distributed. Therefore, in this implementation, the area or surface of the rectangular cross-section 21 at the output ferrule 26 is considered to be and/or to comprise a "two-dimensional surface" within the meaning of the present disclosure.

Preferably, the spatial distribution and/or arrangement of the VCSELs of the VCSEL array(s) 10 shown in figure 2a as well as the spatial distribution and/or arrangement of the distal ends 20 (exit faces) of the fiber bundle 23 shown in figure 2b mounted in the handheld device 1 result in an extended light emitting area, in particular at the exit section 6 of the probe 1, which preferably has between 1 and 10 mm width x 1 to 40 mm length, and offer technical design parameters which enable specific extended laser source parameters matching maximum power exposition (MPE) criteria for laser class 1 or 3R.

Preferably, the divergence of the emerging light beam is custom designed to be between 15° to 35°. Preferably, if a divergence of less than the intrinsic fiber or VCSEL divergence is desired, an optical lens can be provided on the individual VCSEL array or in front of the VCSEL subsystem to adjust the divergence without affecting the concept of the extended source.

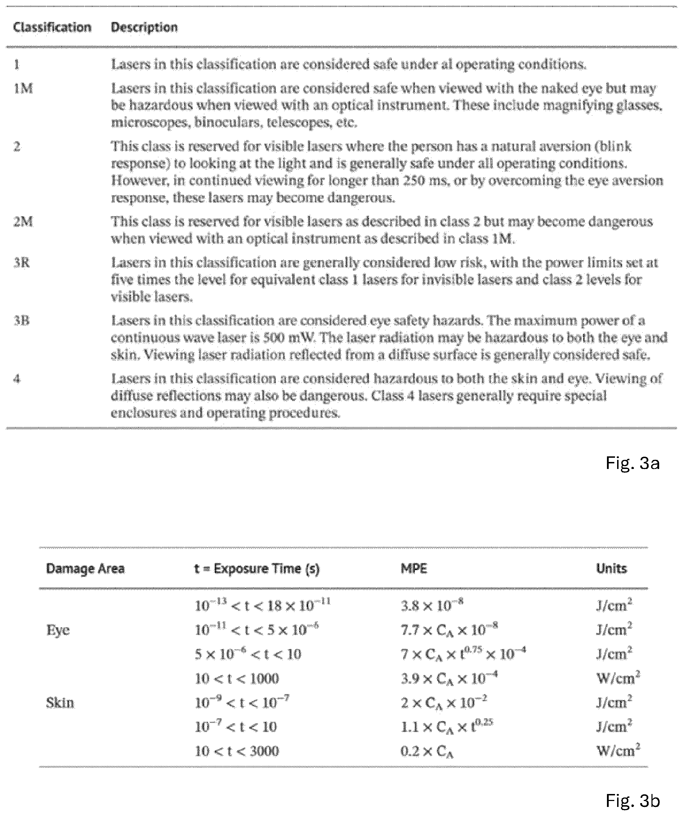

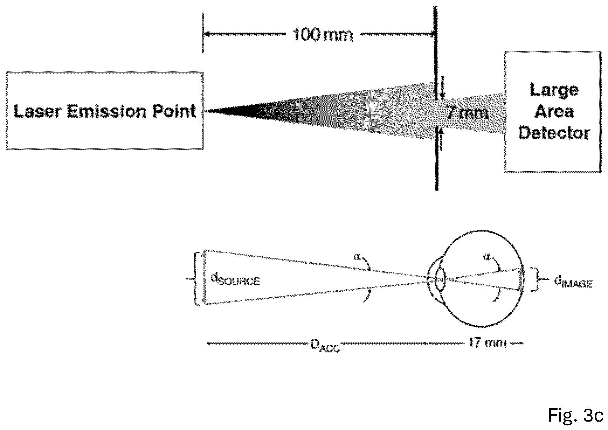

Figure 3a shows a table including definitions of laser classes, including laser class 1 and 3R, according to IEC 60825-1. Figure 3b shows a table including MPE formulas for lasers in the wavelength range between 700 and 1050 nm regarding eye and skin exposure during different exposure times t. Accordingly, an extended source implementation according to present disclosure meeting the requirements of class 1 is considered safe under all operating conditions, and an extended source implementation according to present disclosure meeting the requirements of class 3R is generally considered low risk. To meet the requirements of IEC 60825-1, skin and eye exposure have to be considered. Figure 3c shows schematics of optical systems used to determine the properties of point sources (upper part) and of an extended light source with an artificial eye (lower part), in particular the maximum power exposure (MPE) levels, for a point source (see laser emission point) in the upper part of the figure, and for an extended light source (as with the preferred implementations of an extended source according to present disclosure) having a finite dimension of d_{source} in the lower part of the figure. This dimension d_{source} is imaged to d_{image} at the retina of the artificial eye. Apparently, a standardized "artificial eye" is used to measure the required parameters for IEC 60825-1 to obtain the relevant parameters of the extended light source according to present disclosure.

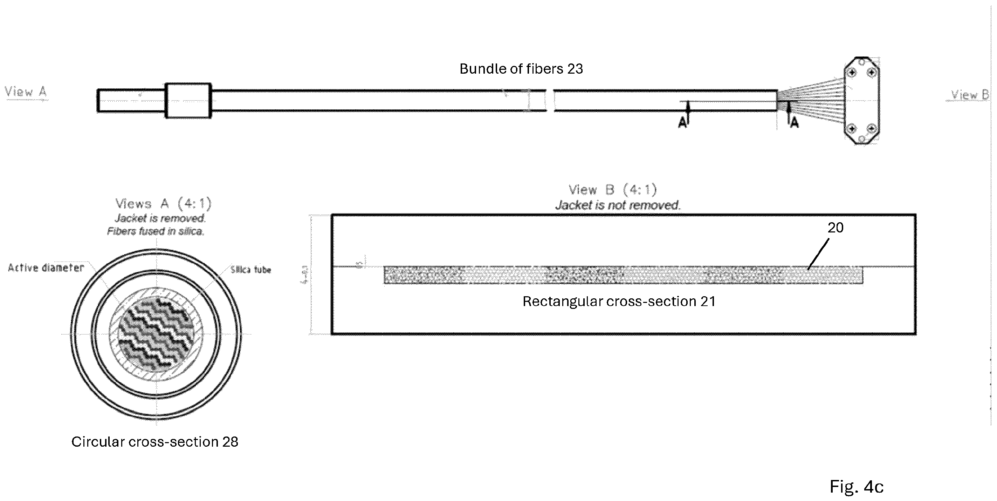

Figure 4 further illustrates preferred details of possible implementations of the extended light source concept, wherein figure 4a and 4b refer to an "internal" VCSEL subsystem 10/15 provided in the handheld device 1, while figure 4c refers to the approach of transmitting light from an "external" laser source 22 provided in the chart 29 via a multi-fiber delivery 23.

Specifically, figure 4a shows a preferred implementation for a laser class 1 subsystem with 12 VCSEL arrays 10 (left) using individual VCSEL arrays 10' (right) for two different wavelengths. The upper part of figure 4b illustrates a far-field superposition of diverging light beams (see side view) of two different wavelengths λ₁, λ₂ emitted by alternatingly arranged individual VCSELs 10 (see top view) to form a total light beam 5 by which the tissue 3 (see figure 1b) is irradiated. The lower part of figure 4b shows photographs of computer screens displaying the results of measurements of the near field (left) and far field (right) of a 940 nm prototype of a VCSEL subsystem using an artificial eye as shown in figure 3c.

Figure 4c shows a schematic representation of a preferred alternative implementation of an extended source with 600 fibers and 24° divergence meeting class 1 or 3R requirements. For example, the proximal end of the bundle of fibers 23 having a preferably circular cross-section 28 is coupled to a YAG-OPO, edge emitting laser diode bar or VCSEL subsystem provided in the cart 29 (see figure 1a). At the distal end of the bundle of fibers 23, which is provided in the probe 1 (see, e.g., fiber holder 4 in figure 1b), the exit faces 20 of the individual fibers are spatially distributed over a preferably rectangular cross-section 21. Providing the laser source in the cart 29 (rather than in the handheld device 1) offers heat dissipation advantages, resulting in a "cold" handheld device 1, in particular because there is no heat dissipation in the handheld device 1.

## Claims

1. An apparatus for sensing properties of biological tissue (3), the apparatus comprising a probe (1), in particular a handheld probe, the probe (1) comprising:
- a plurality of laser radiation emitting elements (10, 20) configured to emit pulses of laser radiation (5), the laser radiation (5) having a predefined wavelength and the pulses of laser radiation (5) having a predefined pulse width and predefined pulse repetition rate,
- an exit section (6), in particular an opening or window, through which the emitted pulses of laser radiation (5) can exit the probe (1) and impinge on the biological tissue (3), and
- at least one detector (7), in particular including piezo, CMUT or PMUT elements, configured to detect signals (8) emanating from the biological tissue (3) in response to the impinging pulses of laser radiation (5) and to convert the detected signals (8) into detector signals from which one or more properties of the biological tissue (3) are derivable,
**characterized in that**
the laser radiation emitting elements (10, 20) are distributed, in particular over a two-dimensional surface (11, 21), whereby for the predefined wavelength, pulse width and pulse repetition rate a radiation energy of the emitted pulses of laser radiation (5) at or close to the exit section (6) of between 1 µJ/mm² and 50 µJ/mm², in particular over an area of 1 to 200 mm² and/or at an emission angle (divergence) of 15° to 45°, is obtained.

2. The apparatus according to claim 1 comprising
- a laser source (22), in particular an Nd:YAG pumped optical parametric oscillator (OPO) and/or an edge-emitting diode laser array and/or a VCSEL array, configured to emit pulsed laser radiation having the predefined wavelength, the predefined pulse width and the predefined pulse repetition rate, and
- a fiber bundle (23) comprising a plurality of single fibers (24) each having an entry face (27) and an exit face (20), wherein the fibers (24) are arranged, in particular bundled, in such a way that, at a proximal end of the fiber bundle (23), the laser radiation emitted by the laser source (22) is or can be coupled into the entry faces (27) of the fibers (24) and, at a distal end of the fiber bundle (23), the exit faces (20) of the fibers (24) are distributed, in particular over the two-dimensional surface (21), and thereby constitute the laser radiation emitting elements.

3. The apparatus according to claim 2, wherein the fiber bundle (23) comprises at least 300, in particular at least 400, at least 500 and/or up to 1500, single fibers (24) and/or the single fibers (24) have a diameter of between 10 and 500 µm, in particular between 100 and 300 µm.

4. The apparatus according to claim 2 or 3, wherein the exit faces (20) of the fibers (24) are distributed over the two-dimensional surface (21) in such a way that on average between 10 and 100, in particular between 20 and 50, exit faces (20) of the fibers (24) are provided per square millimeter (mm²) of the two-dimensional surface (21).

5. The apparatus according to any one of the claims 2 to 4, wherein the repetition rate is between 10 Hz and 100 Hz, in particular between 20 Hz and 30 Hz, in particular if the laser source (22) is an Nd:YAG pumped optical parametric oscillator (OPO), or between 10 Hz and 10 kHz or 20 kHz, preferably between 100 Hz and 10 kHz, in particular if the laser source (22) is at least one VCSEL or a VCSEL array and/or at least one edge-emitting diode laser or an edge-emitting diode laser array.

6. The apparatus according to any one of the claims 2 to 5, wherein the predefined wavelength is between 650 nm and 1200 nm, the predefined pulse width is between 1 ns and 200 ns, the predefined pulse repetition rate is between 10 Hz and 10 kHz, the exit faces (20) of the fibers (24) are distributed over the two-dimensional surface (21) in such a way that on average between 10 and 100 exit faces (20) of the fibers (24) are provided per square millimeter (mm²) of the two-dimensional surface (21), whereby a radiation energy of the emitted pulses of laser radiation (5) at or close to the exit section (5) of between 1 µJ/mm² and 50 µJ/mm² is obtained.

7. The apparatus according to claim 1, wherein the plurality of laser radiation emitting elements is given by a plurality of vertical-cavity surface-emitting lasers (VCSELs) (10) which are distributed, in particular over the two-dimensional surface (11), and configured to emit the pulses of laser radiation (5).

8. The apparatus according to claim 7, wherein the plurality of VCSELs (10) is given by at least 500, in particular at least 1000 or at least 2000, preferably 3000, VCSELs and/or the two-dimensional surface (11) has a size of 0.5 to 4 mm².

9. The apparatus according to claim 7 or 8, wherein the VCSELs are distributed over the two-dimensional surface (11) in such a way that on average between 100 and 1000, in particular between 500 and 600, VCSELs are provided per square millimeter (mm²) of the two-dimensional surface (11).

10. The apparatus according to any one of the claims 7 to 9, wherein the repetition rate is between 100 Hz and 10 kHz, in particular between 500 Hz and 2 kHz.

11. The apparatus according to any one of the claims 7 to 10, wherein the predefined wavelength is between 650 nm and 1200 nm, the predefined pulse width is between 1 ns and 200 ns, the predefined pulse repetition rate is between 100 Hz and 10 kHz, and the VCSELs (10) are distributed over the two-dimensional surface (11) in such a way that on average between 100 and 1000 VCSELs (10) are provided per square millimeter (mm²) of the two-dimensional surface (11), whereby a radiation energy of the emitted pulses of laser radiation (5) at or close to the exit section (6) of between 1 µJ/mm² and 50 µJ/mm² is obtained.

12. The apparatus according to any one of the preceding claims, wherein an irradiance of the emitted pulses of laser radiation (5) at or close to the exit section (6) is between 0.5 mW/mm² and 5 mW/mm², in particular between 1.5 mW/mm² and 3 mW/mm².

13. A system for sensing properties of biological tissue (3), the system comprising:
- an apparatus according to at least one of the preceding claims, and
- a computer system (30), in particular a mobile computer device and/or a computer workstation and/or a cloud computing system, located outside the probe (1) and being in a data communication with the apparatus, the computer system (30) being configured to process the detection signals and/or to derive one or more properties of the biological tissue from the detection signals and/or to reconstruct an image of the biological tissue based on the detection signals and/or to further process an image of the biological tissue which has been reconstructed from the detection signals.
